# EUROPEAN PATENT APPLICATION

(11) **EP 4 180 418 A1**
(43) Date of publication of application: **17.05.2023**
(21) Application number: 20944503.0
(22) Date of filing: 28.12.2020
(51) Int. Cl.: C07C 233/31, A61K 8/68, A61Q 19/00, A61K 31/133, A61P 29/00

(54) **NOVEL SPHINGOLIPID CONTAINING BRANCHED-CHAIN FATTY ACID AND USE THEREOF**

(30) Priority: 10.07.2020 KR 20200085249
(71) Applicant: Solus Biotech Co., Ltd., Iksan-si, Jeollabuk-do 54588 (KR)
(72) Inventor: LEE, Kwanhyoung, Yongin-si, Gyeonggi-do 16858 (KR); KIM, Ara, Yongin-si, Gyeonggi-do 16858 (KR); KIM, Sangchul, Yongin-si, Gyeonggi-do 16858 (KR); HAN, Jihye, Yongin-si, Gyeonggi-do 16858 (KR); KIM, Youngbeom, Yongin-si, Gyeonggi-do 16858 (KR); SEO, Daebang, Yongin-si, Gyeonggi-do 16858 (KR)
(74) Representative: Office Freylinger
(86) International application number: PCT/KR2020/019239
(87) International publication number: WO 2022/010054

(57) **Abstract**

The present invention relates to a topical skin composition comprising a sphingolipid having a branched chain fatty acid as an active ingredient for preventing adsorption of fine dust, improving a skin barrier function, and exhibiting an excellent skin moisture effect, and a preparation method therefor.

## Description

### Technical Field

The present invention relates to a composition for application to the skin and a method for preparing the same, wherein the composition contains as an active ingredient a sphingolipid with a branched chain fatty acid and has effects of preventing fine dust adsorption, enhancing skin barrier function, and having excellent skin moisturizing effects.

### Background Art

The stratum corneum, which is the outermost layer of the skin, is composed of dead corneocytes serving as bricks and intercellular lipid components surrounding the dead corneocytes like cement. The dead corneocytes constituting the stratum corneum contain high-concentration natural moisturizing factors, water-soluble components, which allow the skin to exhibit elasticity as well as maintain an appropriate level of water. In maintaining an appropriate level of water in the stratum corneum, natural moisturizing factors, sebum, and inter-corneocyte lipid components, which are products of epidermal differentiation, and the proper exfoliation of the stratum corneum act as important factors, and an imbalance or lack of these elements may cause dry skin. Recent studies on the correlation between dry skin and ceramides, a main component among the inter-corneocyte lipid components, revealed that the ceramide content and the fatty acid length were shortened in the dry skin. Ceramides are a specific group of sphingolipids including sphingosine, phytosphingosine, or dihydrosphingosine (sphinganine) as a base (sphingoid base) forming an amide bond with a fatty acid. Ceramides are a most important component that accounts for 50 to 60% of lipid components constituting the stratum corneum, the outermost layer, in contact with outside air, and play a main role in keeping the water of the skin to moisturize the skin.

The skin is a part of the body that is directly exposed to the external environment, and serves as a protective layer for protecting important organs of our body from the outside. However, there is an increasing number of cases of skin troubles, such as erythema, edema, and itching, and rapid skin aging since the skin of modern people is constantly exposed to strong ultraviolet light and the pollutants generated during the preparation or disposal of various chemical substances developed with industrialization. The skin troubles not only become aesthetic problems, but cause inflammation responses, the resultant substances from which incidentally cause skin pigmentation and accelerate the collapse of skin elastic fibers to result in even increased skin wrinkles. To prevent lesions and damage caused by fine dust, various preparations for application to the skin have been recently developed, and especially, an industrial attempt has been made to develop preparations for application to the skin that block through covering formation, prevent adhesion through electrostatic attraction, enhance the skin barrier function, and have anti-inflammatory activity. For example, Niraj Mistry et al. have defined skin damage caused by various pollutants, summarized attempts to avoid the damage, and suggested ceramides as promising materials for preventing skin damage caused by fine dust to enhance skin barrier ability.

Vernix caseosa is a viscous, whitish, creamy skin substance coating the entire body of a newborn baby during childbirth. Vernix caseosa, a remnant of the fetal epidermis, is formed at the end of pregnancy, and it has been reported that Vernix caseosa forms a water-resistant membrane in the uterus to protect the fetal skin even in a hot and humid aqueous environment, promotes the maturation of the stratum corneum, prevents bacterial infections, and helps body temperature regulation, antibacterial, cleansing, and hydration actions, and the maturation of the fetal skin even after birth. As low birth-weight infants born before 32 weeks faster than the normal childbirth period are known to have high transepidermal water loss and fast transdermal drug absorption, basic research on structures and biological properties of vernix caseosa was conducted to develop externally applied preparations for skin protection. For example, Oku et al., announced in a literature published in Lipids in 2000, that the structures of ceramides in vernix caseosa are different from those of ceramides of ordinary adult skin, and especially, contain a high proportion of branched chain fatty acids. Korean Patent No. 10-1458108 discloses a novel cosmetic preparation using a vernix caseosa-like lipid complex, but such a preparation has the disadvantage in that the ceramide utilizes an approach to general ceramide 3 known to be contained in adults.

Moreover, ceramides are difficult to apply to cosmetic products due to the problems in solubility thereof and have a deterioration in formulation stability after preparation.

### (Prior Art Document)

(Patent Document 1) KR 10-1458108 B1

### Disclosure of Invention

### Technical Problem

An aspect of the present invention is to implement skin protection ability inherent to vernix caseosa by simulating a vernix caseosa-like sphingolipid component.

Another aspect of the present invention is to provide a composition for application to the skin to prevent fine dust adsorption, increase skin moisture content, and reduce transepidermal water loss.

Another aspect of the present invention is to provide a composition for application to the skin having excellent solubility and formulation stability.

Still another aspect of the present invention is to provide a method for preparing the composition for application to the skin.

Still another aspect of the present invention is to provide a cosmetic or pharmaceutical composition containing the composition for application to the skin.

### Solution to Problem

In accordance with an aspect of the present invention, there is provided a sphingolipid having a branched chain fatty acid and represented by chemical formula 1 below.

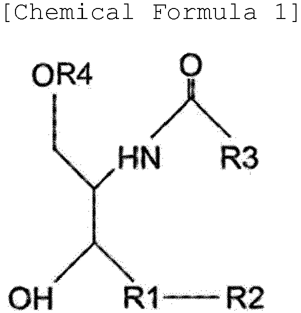

In the chemical formula,
R1 is -CH₂-CH₂, -CH=CH, or -C(H)OH-CH₂;
R2 is a straight or branched chain alkyl, alkenyl, or alkynyl group of 12 to 20 carbon atoms;
R3 is a straight or branched chain alkyl, alkenyl, or alkynyl group of 12 to 24 carbon atoms, which may be optionally substituted with a hydroxyl group and in which two methyl groups are attached to at least one carbon atom; and
R4 is H or a straight or branched chain alkyl or alkenyl group of 1 to 10 carbon atoms.

In accordance with another aspect of the present invention, there is provided a composition for application to the skin, the composition containing the sphingolipid having a branched chain fatty acid and represented by Chemical Formula 1, cholesterol, a fatty acid, a medium chain triglyceride, and a phospholipid.

In accordance with still another aspect of the present invention, there is provided a cosmetic composition containing the above-described composition at 0.00001 to 50 wt% relative to the total weight thereof.

In accordance with still another aspect of the present invention, there is provided a pharmaceutical composition containing the above-described composition at 0.00001 to 50 wt% relative to the total weight thereof.

In accordance with still another aspect of the present invention, there is provided a method for preparing a composition for application to the skin, the method including: (1) preparing a lipid phase by dissolving, in an oil, the sphingolipid having a branched chain fatty acid and represented by Chemical Formula 1 above, cholesterol, a fatty acid, and a phospholipid; (2) preparing an aqueous phase by mixing water with a mixture of a free sphingoid base and an organic acid; and (3) mixing the lipid phase prepared in step (1) and the aqueous phase prepared in step (2) under heating.

### Advantageous Effects of Invention

The branched chain fatty acid-containing sphingolipid of the present invention provides a similar structure to the ceramides of natural vernix caseosa. Therefore, the composition containing the branched chain fatty acid-containing sphingolipid of the present invention, when applied to the skin, can show an anti-inflammatory effect as well as effects of preventing fine dust adsorption, preventing transepidermal water loss, and increasing skin moisture content. Furthermore, the composition has excellent solubility and formulation stability and thus can be applied to cosmetic products and medicinal products.

### Brief Description of Drawings

FIG. 1 shows the silica thin layer chromatography analysis results of acetoxy lanolin ceramide of Example 3 and ceramide 3 as a control group.
FIG. 2 shows the high performance liquid chromatograph analysis results of acetoxy lanolin ceramide of Example 3.
FIG. 3 shows the Turbiscan test results of compositions of Example 5 and Comparative Examples 3 and 4.
FIG. 4 shows a branched chain fatty acid ceramide-containing cream formulation of Example 6 and a polarization microscopic image thereof.
FIG. 5 shows the fine dust adsorption prevention test results through the use of a branched chain fatty acid ceramide-containing cream formulation of Example 6.
FIGS. 6A and 6B shows the transepidermal water loss prevention test results and skin moisture content increase test results through the use of a formulation employing a branched chain fatty acid-containing ceramide of Example 6.
FIG. 7 shows the erythema relief test results through the use of a formulation employing a branched chain fatty acid-containing ceramide of Example 6.

### Best Mode for Carrying out the Invention

Hereinafter, the present invention will be described in detail.

The present inventors, while making an intensive endeavor to develop a novel composition based on characteristics of natural vernix caseosa having excellent efficacy in enhancing the skin barrier function and skin moisturizing effect, developed a composition containing as an active ingredient a novel sphingolipid having a long chain and branched chain fatty acid on the basis of the fact that the ceramide content and the fatty acid length are short in the dry skin and the structure of ceramides in vernix caseosa are different from those of ceramides of ordinary adult skin, and the present inventors verified that such a composition, when applied to the skin, showed an anti-inflammatory effect as well as effects of preventing fine dust adsorption, preventing transepidermal water loss, and increasing the skin moisture content, and then completed the present invention.

As used herein, the term "sphingolipid" refers to a generic term of lipids having a long chain sphingoid base, and encompasses sphingoglycolipids, sphingophospholipids, or ceramides.

Sphingolipids have, as a common structure, a ceramide structure with an irregular chain length in which a long chain fatty acid is bound to an amino group of a sphingoid via an acid-amide bond.

Ceramides have an acid-amide bond structure of a fatty acid and a base (sphingoid base), which is sphingosine, phytosphingosine, or dihydrosphingosine (sphinganine) .

### <Sphingolipid having branched chain fatty acid>

An aspect of the present invention is directed to a sphingolipid having a branched chain fatty acid and represented by chemical formula 1 below.

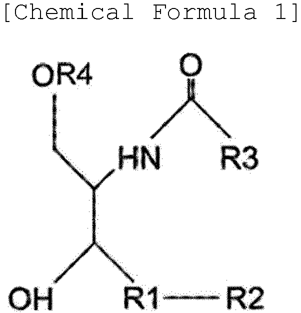

In the chemical formula, R1 is -CH₂-CH₂, -CH=CH, or -C(H)OH-CH₂;
R2 is a straight or branched chain alkyl, alkenyl, or alkynyl group of 12 to 20 carbon atoms;
R3 is a straight or branched chain alkyl, alkenyl, or alkynyl group of 12 to 24 carbon atoms, which may be optionally substituted with a hydroxyl group and in which two methyl groups are attached to at least one carbon atom; and
R4 is H or a straight or branched chain alkyl or alkenyl group of 1 to 10 carbon atoms.

The sphingolipid used in the present invention contains a branched chain fatty acid.

The term "branched chain fatty acid" refers to a fatty acid in which a branch chain (methyl group or the like) is formed on the carbon chain of a fatty acid.

In the present invention, the branched chain fatty acid may be a straight or branched chain alkyl, alkenyl, or alkynyl group of 12 to 20 carbon atoms in which two methyl groups are attached to at least one carbon atom, in R3.

As such, the formation of the branched chain fatty acid by attaching two methyl groups to at least one carbon atom in the carbon chain of the fatty acid can exhibit a skin protective covering forming effect.

The sphingolipid of the present invention may be a sphingolipid having a single branched chain fatty acid or a complex branched chain fatty acid. The single branched chain fatty acid refers to one in which two methyl groups are attached to one carbon atom, and the complex branched chain fatty acid refers to one in which two methyl groups are attached to two or more carbon atoms.

According to an embodiment of the present invention, R3 may be a straight or branched chain alkyl, alkenyl, or alkynyl group of 12 to 24 carbon atoms, preferably 15 to 22 carbon atoms.

R3 may be derived from at least one oil selected from the group consisting of palm, sunflower, canola, olive, coconut, soybean, horse, cattle, sheep, bee, fish, crustacean, and human, but is not limited thereto.

In addition, R3 may be optionally substituted with a hydroxyl group.

R4 is H or a straight or branched chain alkyl or alkenyl group of 1 to 10 carbon atoms.

The sphingolipid having a branched chain fatty acid and represented by Chemical Formula 1 may be synthesized by an enzyme that specifically dehydrates an acid-amide bond of a sphingoid and a fatty acid, or a microorganism having enzyme producing ability, or a synthetic method.

The sphingolipid having a branched chain fatty and represented by Chemical Formula 1 may have a melting point of 10-100° C. In an example of the present invention, branched chain stearoyl ceramide had a much lower melting point than a ceramide containing no branch chain (see Table 1). A lower melting point allows the consumption of less energy, and therefore such a ceramide can be easily applied to a formulation.

In addition, the sphingolipid having a branched chain fatty acid and represented by Chemical Formula 1 has high solubility, and thus was not recrystallized even when cooled to room temperature after heating (see Table 2). A high solubility improves the stability in formulations and thus causes no precipitation.

### <Composition for application to skin>

The present invention is directed to a composition for application to the skin, the composition containing a sphingolipid having a branched chain fatty acid and represented by Chemical Formula 1, cholesterol, a fatty acid, a medium chain triglyceride, and a phospholipid.

In an embodiment of the present invention, the sphingolipid having a branched chain fatty acid and represented by Chemical Formula 1 may be used at 0.01 to 6 wt%, preferably 0.1 to 4 wt% relative to the total weight of the composition.

Less than 0.01 wt% of the sphingolipid may be insufficient to prevent fine dust adsorption, prevent transepidermal water loss, increase the skin moisture content, and exert an anti-inflammatory effect, and more than 6 wt% of the sphingolipid may have lowered stability in formulations.

As the cholesterol used in the present invention, any cholesterol substance known in the art may be used without limitation, and examples thereof may include synthetically and naturally-derived cholesterols, cholesteryl alkylates, such as cholesteryl stearate and cholesteryl isostearate, prepared by esterification of cholesterol, and cholesteryl glutamate prepared by esterification of an amino acid with cholesterol. The cholesterol may be contained at 0.1 to 5 wt%, preferably 0.5 to 2 wt% relative to the total weight of the composition.

In the present invention, as the fatty acid, any common component known in the art may be used without limitation, and for example, a straight chain saturated fatty acid of 12 to 30 carbon atoms, preferably 18 to 26 carbon atoms, may be used. For example, the fatty acid may be at least one selected from the group consisting of lauric acid, myristic acid, palmitic acid, stearic acid, oleic acid, arachidic acid, behenic acid, lignoceric acid, cerotic acid, arachidonic acid, eicosapentaenoic acid, erucic acid, branched chain lauric acid, branched chain myristic acid, branched chain palmitic acid, branched chain stearic acid, branched chain oleic acid, branched chain arachidic acid, branched chain behenic acid, and branched chain lignoceric acid. The fatty acid may be contained at 0.1 to 8 wt%, preferably 0.5 to 6 wt% relative to the total weight of the composition.

In the present invention, a phospholipid (lecithin), which is a cellular component, is a main component constituting cell membranes. Phospholipids play an important role in enzymatic reaction regulation by providing environments of actions of various enzymes and play a variety of roles in maintaining biological homeostasis by serving also as secondary transmitters in cells by various stimuli. As the phospholipid, any common component known in the art may be used without limitation, and a non-limiting example thereof may be at least one selected from the group consisting of hydrogenated lecithin, hydrogenated phosphatidylcholine, phospholipids, hydrogenated lysophosphatidylcholine, hydrogenated lysolecithin, hydroxylated lecithin, and unsaturated lecithin. The phospholipid may be contained at 0.1 to 5 wt%, preferably 0.5 to 4 wt% relative to the total weight of the composition.

In the present invention, the medium chain triglyceride may help an emollient and moisture bind to the skin. Examples of the medium chain triglyceride may include caprylic/capric triglyceride, caprylic/capric triglyceride, and the like. The medium chain triglyceride may be contained at 0.1 to 5 wt%, preferably 0.5 to 5 wt% relative to the total weight of the composition.

In addition, the composition may further contain a free sphingoid base, an organic acid, and water as balance to a total of 100 wt%.

The free sphingoid base used in the present invention is an intermediate in the biosynthetic pathway of a ceramide and may serve to supplement a deficient ceramide component or enable skin cells to directly synthesize the ceramide when applied to the skin. Preferably, the free sphingoid base may be at least one selected from the group consisting of phytosphingosine, sphingosine, and sphinganine, and more preferably may be phytosphingosine. The free sphingoid base may be contained at 0.1 to 3 wt%, preferably 0.5 to 2 wt% relative to the total weight of the composition.

In the present invention, as the organic acid, any common component known in the art may be used without limitation, and examples thereof may include carboxylic acid, sulfonic acid, phosphoric acid, fumaric acid, lactic acid, citric acid, butyric acid, formic acid, acetic acid, propionic acid, and the like. The organic acid may be contained at 0.05 to 0.3 wt% relative to the total weight of the composition.

According to an embodiment of the present invention, the composition contains a lipid phase and an aqueous phase, wherein the lipid phase may contain 0.1 to 6 wt% of the sphingolipid containing a branched chain fatty acid and represented by Chemical Formula 1, 0.1 to 5 wt% of cholesterol, 0.1 to 8 wt% of the fatty acid, 0.1 to 5 wt% of the medium chain triglyceride, and 0.1 to 5 wt% of the phospholipid, and the aqueous phase may contain 0.1 to 3 wt% of the free sphingoid base, 0.05 to 0.3 wt% of the organic acid, and water as balance to a total of 100 wt%.

According to an embodiment of the present invention, the composition for application to the skin may further contain at least one ceramide selected from the group consisting of ceramides 1, 2, 3, 4, 5, 6, 7, 8, and 9. As a result, the composition for application to the skin may contain a combination of a free sphingoid base and a ceramide. As a result, the composition for application to the skin may be composed of two types of ceramides having head groups with different hydrophilic moieties and thus can have enhanced barrier properties.

According to an embodiment of the present invention, the composition for application to the skin may have uses for preventing fine dust adsorption, improving skin moisturization, enhancing skin barriers, exerting an anti-inflammatory effect, healing wounds, relieving human body wrinkles, or modifying the skin.

The composition for application to the skin of the present invention can prevent the adsorption of fine dust having a size of 0.1 to 100 *µ*m, preferably nitrates, sulfates, carbons, and other minerals, through the filling of the stratum corneum.

The composition for application to the skin of the present invention may be used as a composition for external application to the skin, which contains a branched chain fatty acid-containing sphingolipid having a similar structure to the ceramides of natural vernix caseosa and thus implement skin protection ability inherent to vernix caseosa.

Ceramides are difficult to apply to cosmetic products due to their solubility problem and have a deterioration in formulation stability after preparation.

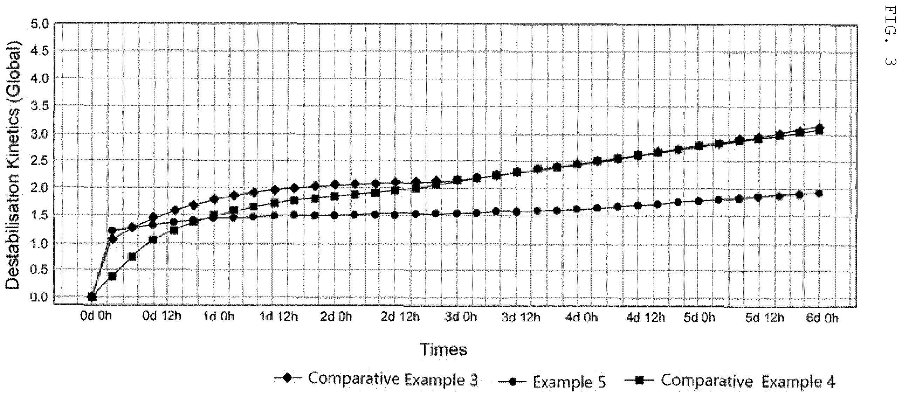

The composition of the present invention is stable and can form a Maltese-cross liquid crystal when applied to a final formulation. Specifically, the Maltese-cross liquid crystal may have a single layer or multiple layers, and preferably multiple layers. As a test result, in a cream formulation prepared using the branched chain fatty acid ceramide of the present invention, the phase separation and crystal precipitation were not observed, the formulation stability was shown, and the multi-lamella Maltese-cross liquid crystal was confirmed, and favorable results in view of uniformity of a liquid crystal phase was shown (see Tables 3 and 4 and FIG. 3).

The sphingolipid-containing composition of the present invention has a similar structure and composition to vernix caseosa, and can have improved skin absorption and enhanced skin permeation by forming a liquid crystal when applied to a formulation. In general, a liquid crystal is frequently used by introduction into cosmetic products, wherein the liquid crystal is dissolved in an oil phase and then formed in the interface by oil diffusion, contributing to improved skin moisturization. Since the liquid crystal is structurally similar to skin lipids, the liquid crystal component is well spread on the skin surface compared with other components, resulting in smooth and soft skin. In addition, the liquid crystal collects a predetermined amount of bounded water, and such water was not well evaporated unlike free water. That is, the bounded water thus collected stays like a liquid crystal on the skin, resulting in a contribution to an increased rate of skin moisture content.

According to the present invention, a sphingolipid having a branched chain fatty acid is used to form a Maltese-cross liquid crystal covering having a similar composition to vernix caseosa sphingolipids, and the formed liquid crystal covering allows a high moisturization effect due to a long-lasting effect, leading to heathier and smoother skin.

An embodiment of the present invention provides a cosmetic composition containing the composition for application to the skin at 0.00001 to 50 wt% relative to the total weight of the cosmetic composition.

The cosmetic composition may be used to prevent fine dust adsorption, improve skin moisturization, enhance skin barriers, relieve human body wrinkles, and modify the skin.

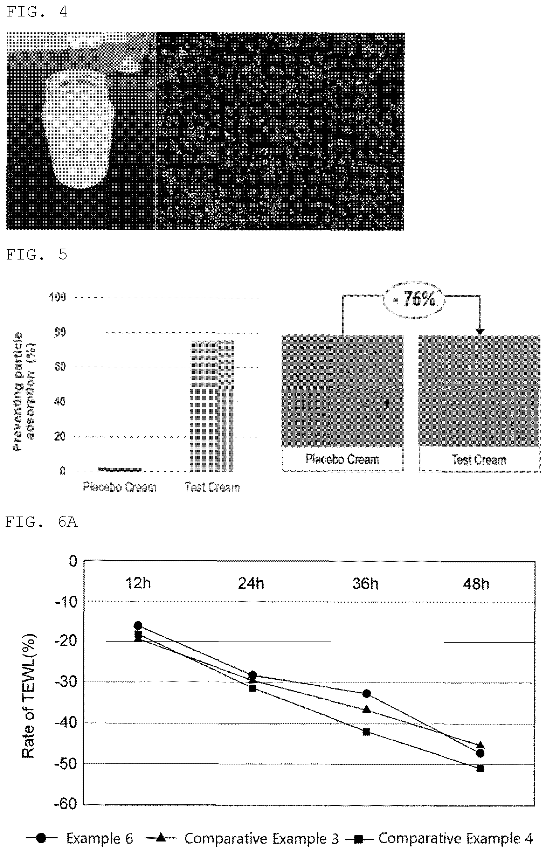

The composition containing the branched chain fatty acid-containing sphingolipid of the present invention, when applied to the skin, can prevent fine dust adsorption, prevent transepidermal water loss, increase the skin moisture content, and show an anti-inflammatory effect (see Tables 5 to 7 and FIGS. 5 to 7 below).

The cosmetic composition of the present invention contains a branched chain fatty acid-containing sphingolipid having a similar structure to the ceramides of natural vernix caseosa, and thus can implement the skin protecting ability inherent to vernix caseosa. Therefore, the cosmetic composition of the present invention may be used for protecting the skin or improving skin moisturization.

The cosmetic composition of the present invention contains ingredients that are commonly used in cosmetic compositions, in addition to the above-described ingredients as active ingredients. For example, the cosmetic composition of the present invention contains a carrier and common adjuvants, such as antioxidants, stabilizers, solubilizers, vitamins, pH adjusters, colorants, flavoring agents, pigments, and flavors that are commonly known in the art.

The cosmetic composition of the present invention may be prepared in any formulation that is commonly prepared in the art, and for example, the cosmetic composition of the present invention may be formulated into a solution, a suspension, an emulsion, a paste, a gel a cream, a lotion, a powder, a soap, a surfactant-containing cleanser, an oil, a powder foundation, an emulsion foundation, a wax foundation, and a spray, but is not limited thereto. More specifically, the cosmetic composition of the present invention may take a formulation of a soft lotion (skin), an astringent lotion, a nourishing lotion (milk lotion), a nourishing cream, a massage cream, an essence, an eye cream, a cleansing cream, a cleansing foam, a cleansing water, a pack, a spray, or a powder. In cases where the formulation of the present invention is a paste, a cream, or a gel, an animal oil, a plant oil, wax, paraffin, starch, tragacanth, a cellulose derivative, polyethylene glycol, silicone, bentonite, silica, talc, zinc oxide, or the like may be used as a carrier component. In cases where the formulation of the present invention is a powder or a spray, lactose, talc, silica, aluminum hydroxide, calcium silicate, or a polyamide powder may be used as a carrier component, and particularly, in cases where the formulation is a spray, the spray may further contain a propellant, such as chlorofluorohydrocarbon, propane/butane, or dimethyl ether. In cases where the formulation of the present invention is a solution or an emulsion, a solvent, a solubilizer, or an emulsifier may be used as a carrier component, and examples thereof include water, ethanol, isopropanol, ethyl carbonate, ethyl acetate, benzyl alcohol, benzyl benzoate, propylene glycol, 1,3-butyl glycol oil, a glycerol aliphatic ester, polyethylene glycol, or a sorbitan fatty acid ester.

An embodiment of the present invention provides a pharmaceutical composition containing the composition for application to the skin of the present invention at 0.00001 to 50 wt% relative to the total weight of the pharmaceutical composition.

The pharmaceutical composition may be used to prevent or treat inflammation, improve skin moisturization, enhance skin barriers, relieve human body wrinkles, and modify the skin.

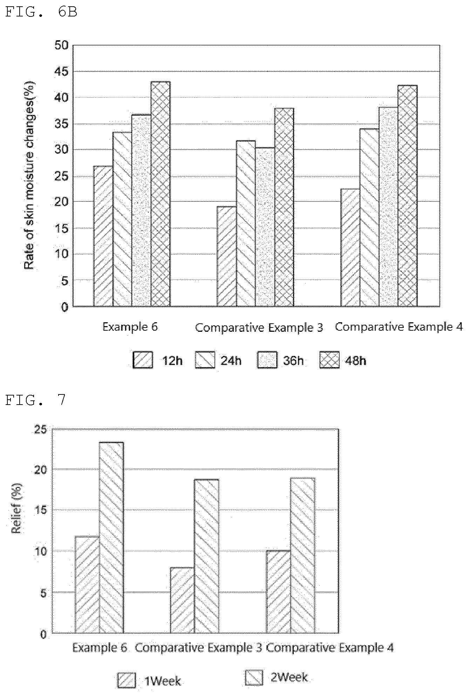

The composition containing the branched chain fatty acid-containing sphingolipid of the present invention, when applied to the skin, could prevent transepidermal water loss, increase the skin moisture content, and show an anti-inflammatory effect (see Tables 5 to 7 and FIGS. 6 and 7 below).

### <Method for preparing composition for application skin>

A method for preparing a composition for application to the skin according to an embodiment of the present invention includes the steps of: (1) preparing a lipid phase by dissolving, in an oil, the sphingolipid having a branched chain fatty acid and represented by Chemical Formula 1, cholesterol, a fatty acid, and a phospholipid; (2) preparing an aqueous phase by mixing water with a mixture of a free sphingoid base and an organic acid; and (3) mixing the lipid phase prepared in step (1) and the aqueous phase prepared in step (2) under heating.

According to an embodiment of the present invention, in step (1), the sphingolipid having a branched chain fatty acid and represented by Chemical Formula 1 may be used at 0.01 to 6 wt%, preferably 0.1 to 4 wt% relative to the total weight of the composition.

According to an embodiment of the present invention, in step (1), the cholesterol may be cholesterol, cholesteryl stearate, cholesteryl isostearate, cholesteryl glutamate, or the like. The cholesterol may be used at 0.1 to 5 wt%, preferably 0.5 to 2 wt% relative to the total weight of the composition.

According to an embodiment of the present invention, in step (1), the fatty acid may be at least one selected from the group consisting of lauric acid, myristic acid, palmitic acid, stearic acid, oleic acid, branched chain lauric acid, branched chain myristic acid, branched chain palmitic acid, branched chain stearic acid, and branched chain oleic acid. The fatty acid may be used at 0.1 to 8 wt%, preferably 0.5 to 6 wt% relative to the total weight of the composition.

According to an embodiment of the present invention, in step (1), the phospholipid may be at least one selected from the group consisting of hydrogenated lecithin, hydrogenated phosphatidylcholine, phospholipids, hydrogenated lysophosphatidylcholine, hydrogenated lysolecithin, hydroxylated lecithin, and unsaturated lecithin. The phospholipid may be used at 0.1 to 5 wt%, preferably 0.5 to 4 wt% relative to the total weight of the composition.

According to an embodiment of the present invention, in step (1), the medium chain triglyceride may be further dissolved in the oil. Examples of the medium chain triglyceride may include caprylic/capric triglyceride, and the like. The medium chain triglyceride may be used at 0.1 to 5 wt%, preferably 0.5 to 5 wt% relative to the total weight of the composition.

According to an embodiment of the present invention, in step (2), the free sphingoid base may be at least one selected from the group consisting of sphingosine, phytosphingosine, and sphinganine. Preferably, the free sphingoid base may be phytosphingosine. The free sphingoid base may be used at 0.1 to 3 wt%, preferably 0.5 to 2 wt% relative to the total weight of the composition.

According to an embodiment of the present invention, in step (2), the organic acid may be carboxylic acid, sulfonic acid, phosphoric acid, fumaric acid, lactic acid, citric acid, butyric acid, formic acid, acetic acid, propionic acid, or the like. The organic acid may be used at 0.05 to 0.3 wt% relative to the total weight of the composition.

According to an embodiment of the present invention, in step (3), the heating may be performed at 60 to 90°C.

According to an embodiment of the present invention, in step (3), the mixing may be accompanied by stirring.

### Mode for Carrying out the Invention

Hereinafter, the present invention will be described in detail with reference to examples and comparative examples. These examples are provided only for the purpose of illustrating the present invention in more detail, and it would be apparent to a person skilled in the art that the scope of the present invention is not limited by these examples.

### Example 1: Preparation of fatty acid

Lanolin fat obtained from wool was separated into an alcohol moiety and a glycerol moiety of lanolin by treatment with ethanol and caustic soda, and then subjected to treatment with chloric acid and fractionation with hexane, thereby obtaining the alcohol moiety as a fatty acid.

### Example 2: Preparation of acetoxy lanolin fatty acid

The lanolin fatty acid prepared in Example 1 was dissolved in ethyl acetate and then treated with triethyl amine and acetic anhydride. After the completion of reaction, the lanolin fatty acid was phase-separated once with 3 N HCl and three times with saline, and then residual acetic acid was removed. Then, the upper layer was treated with sodium sulfate, followed by concentration and then drying.

### Example 3: Preparation of acetoxy lanolin ceramide

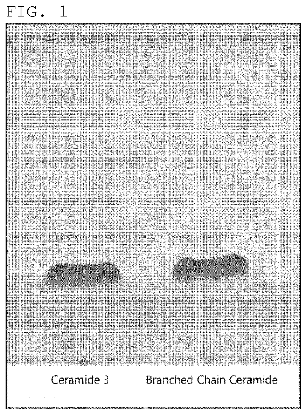

The acetoxy lanolin fatty acid prepared in Example 2 was dissolved in ethyl acetate, and then sodium methoxide and phytosphingosine were added thereto. After reaction at 50°C for 2 hours, the corresponding reaction solution was cooled to 5°C and filtered to obtain a white filtrate. The white filtrate was dried, and then was analyzed by silica thin layer chromatography (development conditions: chloroform + methanol + formic acid = 190:15:1, color development with 10% sulfuric acid) to confirm an acetoxy lanolin ceramide (FIG. 1). Ceramide 3 was used as a control group.

### Test Example 1: High performance liquid chromatography analysis

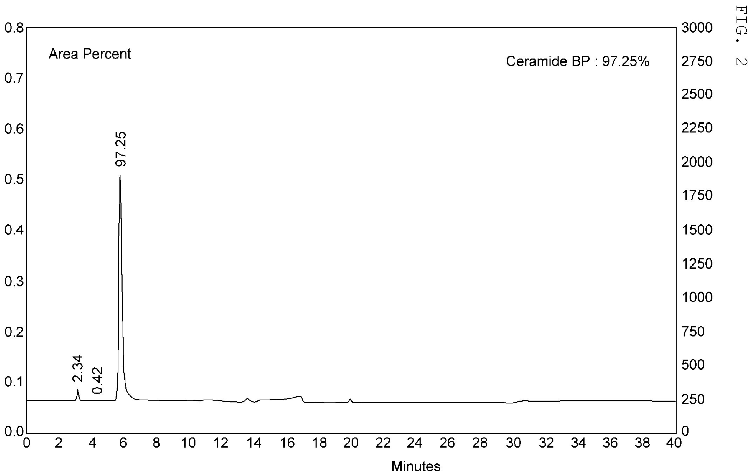

The acetoxy lanolin ceramide prepared in Example 3 was dissolved in methanol and then treated with KOH. The KOH reaction solution was cooled to 5° C and then filtered to obtain a white filtrate. The white filtrate thus obtained was analyzed by high performance liquid chromatography (mobile phase: chloroform + methanol = 96:4, normal phase stationary phase, light scattering detector) to confirm a ceramide peak with an area value of 96% (FIG. 2).

### Example 4: Preparation of branched chain stearoyl ceramide

After 3.5 g of branched chain stearic acid was dissolved in 20 ml of ethyl acetate, 3 g of tosyl chloride and 1 g of 4-dimethylaminopyridine (DMAP) were added, followed by reaction for 0.5 hours, and then 4 g of phytosphingosine (R1=(CH₂-CH₂), R2=(C₁₃H₂₇), R4=(H)) was added. The reaction was conducted for 2 hours after the addition of phytosphingosine, and then the reaction solution was cooled to 10°C and filtered to obtain 4 g of a white filtrate. The white filtrate thus obtained was dried and analyzed by liquid chromatography to confirm a ceramide peak with an area value of 98%.

### Comparative Example 1: Preparation of stearoyl ceramide

A ceramide was prepared by the same method as in Example 4 except that stearic acid without a branched chain was used.

### Comparative Example 2: Preparation of oleoyl ceramide

A ceramide was prepared by the same method as in Example 4 except that oleic acid having a double bond but not a branched chain was used.

### Test Example 2: Melting point

The ceramides of Example 4, Comparative Example 1, and Comparative Example 2 were measured for the melting point by using a capillary tube, and the results are shown in Table 1 below.

**TABLE 1**

| Classification | Branched chain stearoyl ceramide (Example 4) | Stearoyl ceramide (Comparative Example 1) | Oleoyl ceramide (Comparative Example 2) |
|---|---|---|---|
| Melting point (°C) | 94.7 | 115.4 | 105.4 |

As shown in Table 1, the melting point of the branched chain stearoyl ceramide of Example 4 was much lower than those of the ceramides of Comparative Example 1 and Comparative Example 2.

### Test Example 3: Solubility

The ceramides of Example 4, Comparative Example 1, and Comparative Example 2 were mixed with oils and polyols at 0.1 to 1.0 wt% relative to the solvent substance, and then heated with stirring at 80°C and then cooled to room temperature. The comparison of solubility was conducted and the results are shown in Table 2.

**TABLE 2**

| Solvent | Branched chain stearoyl ceramide (Example 4) | | | Stearoyl ceramide (Comparative Example 1) | | | Oleoyl ceramide (Comparati ve Example 2) | |
|---|---|---|---|---|---|---|---|---|
| INCI Name | 0.1% | 0.5% | 1.0% | 0.1% | 0.5% | 1.0% | 0.1% | 0.5% |
| Dipropylene glycol | ++ | ++ | ++ | + | - | - | + | - |
| Propylene glycol | ++ | ++ | ++ | - | - | - | - | - |
| Butylene glycol | ++ | ++ | ++ | + | + | - | + | + |
| Glycerin | - | - | - | - | - | - | - | - |
| Caprylic/capric triglyceride | + | - | - | - | - | - | - | - |
| Cetyl ethyl hexanoate | ++ | + | - | - | - | - | - | - |
| Macadamia ternifolisa seed oil | + | - | - | - | - | - | - | - |

| | | | | | | | | |
|---|---|---|---|---|---|---|---|---|
| (-): Non-soluble (+): Soluble (recrystallization occurred after cooling to room temperature) (++): Very soluble (recrystallization did not occur after cooling to room temperature) | | | | | | | | |

As shown in Table 2, the solubility of the branched chain stearoyl ceramide of Example 4 was much higher than those of the ceramides of Comparative Example 1 and Comparative Example 2.

### Example 5: Preparation and stability of composition

The ceramides of Example 4, Comparative Example 1, and Comparative Example 2 were used to prepare compositions of Example 5, Comparative Example 3, and Comparative Example 4, which were then dispersed in water, and then subjected to Turbiscan analysis at 45°C, and the results are shown in FIG. 3.

**TABLE 3**

| Classifi cation | Raw material | Content (%) | | |
|---|---|---|---|---|
| | | Branched chain stearoyl ceramide (Example 4) | Stearoyl ceramide (Comparative Example 1) | Oleoyl ceramide (Comparative Example 2) |
| Aqueous phase | Water | To 100 | To 100 | To 100 |
| | Phytosphing osine | 1.5 | 1.5 | 1.5 |
| | Organic acid | 0.2 | 0.2 | 0.2 |
| Oil phase | Medium chain triglycerid e | 3 | 3 | 3 |
| | Cholesterol | 2 | 2 | 2 |
| | Stearic acid | 2.5 | 2.5 | 2.5 |
| | Lecithin | 3.5 | 3.5 | 3.5 |
| | Ceramide | 3.5 | 3.5 | 3.5 |
| | Oleic acid | 2.5 | 2.5 | 2.5 |
| Preserva tive | Phenoxy ethanol | 0.2 | 0.2 | 0.2 |

As a result, as shown in FIG. 3, the composition containing the branched chain stearic acid ceramide prepared in Example 5 was stable.

### Example 6: Preparation, stability, and liquid crystal-forming ability of cream formulation employing composition

A cream formulation of Example 6 shown in Table 4 was prepared by using the branched chain fatty acid ceramide of Example 4, and then analyzed by a polarization microscope, and the results are shown in FIG. 4.

**TABLE 4**

| NO | Trade name | INCI name | Ceramide Content of liquid crystal cream ingredient (wt%) |
|---|---|---|---|
| 1 | Lanette-O | Cetostearyl alcohol | 3.00 |
| 3 | GMS 205 | Glyceryl stearate | 2.00 |
| 4 | Oliwax LC | Palmitate, Sorbitan; and Palmitate, Sorbitan O) | 2.00 |
| 5 | Olivem 1000 | Cetearyl Olivate | 0.50 |
| | | Sorbitan Olivate | |
| 6 | Branched chain fatty acid ceramide | | 1.50 |
| 7 | Squalane | Squalane | 5.00 |
| 8 | Silicone 200f/100s | Dimethicone | 0.30 |
| 9 | D.I Water | Water | 77.08 |
| 10 | EDTA-2Na | Disodium EDTA | 0.02 |
| 11 | KMO-6 | 1,2-Hexanediol | 2.00 |
| 12 | 1,3BG | Butylene glycol | 3.00 |
| 13 | Glyserine | Glycerin | 5.00 |
| 14 | Keltrol F | Xanthan gum | 0.10 |
| Total | | | 100.00 |

As confirmed in FIG. 4, the cream formulation prepared using the branched chain fatty acid ceramide prepared in Example 4 had formulation stability since phase separation and crystal precipitation were not observed therein. The cream formulation prepared using the branched chain fatty acid ceramide in Example 4 was a Maltese-cross liquid crystal phase, which was a multilamellar type, and was favorable in view of uniformity of the liquid crystal phase.

### Test Example 4: Test on fine dust adsorption prevention ability

The branched chain fatty acid ceramide-containing cream formulation prepared in Example 6 was tested for fine dust adsorption prevention ability, and the results are shown in FIG. 5.

As confirmed in FIG. 5, the branched chain fatty acid ceramide-containing cream formulation prepared in Example 6 showed a 76% reduction in fine dust adsorption compared with the placebo cream.

### Test Example 5: Tests on transepidermal water loss preventing effect and skin moisture content increasing effect

The branched chain fatty acid ceramide-containing formulation prepared in Example 6 was analyzed for transepidermal water loss to investigate a transepidermal water loss preventing effect (FIG. 6A).

Specifically, in a constant temperature and constant humidity chamber at 22°C and 45% relative humidity, 20 test subjects were measured for water evaporation amount, wherein the upper arm part was tape-stripped to weaken the skin barrier, and then the cream employing the branched chain fatty acid ceramide prepared in Example 4 and the compositions of Comparative Examples 3 and 4 employing the ceramides prepared in Comparative Examples 1 and 2 were applied thereon at a predetermined amount (0.03 g/16 cm²) and then well spread. TEWL was measured by Tewameter TM 210, an evaporatimeter of Courage+Khazaka (C-K) (Cologne, Germany). After the application, the water evaporation amount was measured five times at intervals of 1 minute, every 12 hours, and average values were obtained. The results are shown in Table 5 and FIG. 6A.

**TABLE 5**

| Classification (water evaporation amount) | Example 6 | Comparative Example 3 | Comparative Example 4 |
|---|---|---|---|
| After 0 hours | 11.3714.53 | 11.3412.32 | 11.9911.70 |
| After 12 hours | 9.5411.85 | 9.1812.17 | 9.8111.30 |
| After 24 hours | 8.1812.76 | 8.02±1.95 | 8.2412.85 |
| After 36 hours | 7.67±1.83 | 7.1912.27 | 6.9711.54 |
| After 48 hours | 6.0412.01 | 6.21±1.15 | 5.8911.24 |

As confirmed in Table 5 and FIG. 6A, the branched chain fatty acid ceramide-containing formulation prepared in Example 6 showed a transepidermal water loss preventing effect. In addition, the branched chain fatty acid ceramide-containing formulation prepared in Example 6 was analyzed for skin moisture content to investigate a skin moisture content increasing effect (FIG. 6B).

Specifically, for the measurement of skin moisture content, a total of 20 test subjects were subjected to application of the branched chain fatty acid ceramide-containing formulation prepared in Example 6 twice a day, wherein the moisture content was measured a total of three times on day 3, day 5, day 8, and day 10, and average values were obtained to measure the change in moisture content. Comparative Examples 3 and 4 as control groups were also measured for the moisture content change by the same method. The moisture content was measured by Corneometer (CM825, Courage-Khazaka Electronic GmbH, Germany) of Courage+Khazaka (C-K) (Cologne, Germany) in a constant temperature and constant humidity chamber at 22°C and 45% relative humidity. The results are shown in Table 6 and FIG. 6B.

**TABLE 6**

| Classification (skin moisture content) | Example 6 | Comparative Example 3 | Comparative Example 4 |
|---|---|---|---|
| After 0 hours | 35.91±6.73 | 35.14±6.99 | 34.35±6.65 |
| After 12 hours | 38.38±6.58 | 37.97±8.75 | 37.39±9.86 |
| After 24 hours | 40.33±9.58 | 40.94±7.33 | 40.84±9.11 |
| After 36 hours | 41.36±10.27 | 41.54±10.78 | 42.13±8.68 |
| After 48 hours | 43.24±8.71 | 43.94±7.33 | 43.39±7.21 |

As confirmed in Table 6 and FIG. 6B, the branched chain fatty acid ceramide-containing formulation prepared in Example 6 showed a skin moisture content increasing effect.

### Test Example 6: Test on erythema relieving effect

The branched chain fatty acid ceramide-containing formulation prepared in Example 6 was clinically tested for erythema relieving effect.

Specifically, for erythema measurement, a total of 10 test subjects were subjected to application of the branched chain fatty acid ceramide-containing formulation prepared in Example 6 twice a day, and measured before use (Week 0), 1 week after use, and 2 weeks after use by an instrument. The skin erythema index on the cheek area was measured using Mexameter MX 18 of Courage+Khazaka (C-K) (Cologne, Germany). The results are shown in Table 7 and FIG. 7.

**TABLE 7**

| Classificatio n (Erythema) | Example 6 | Comparative Example 1 | Comparative Example 2 |
|---|---|---|---|
| Week 0 | 289.93±6.14 9 | 294.2±20.316 | 261.53±10.82 |
| Week 1 | 256.4±8.500 | 270.87±20.52 9 | 236.2±11.578 |
| Week 2 | 222.8±8.051 | 238.8±18.413 | 213.47±11.51 8 |

As can be seen from Table 7 and FIG. 7, the ceramide-containing formulation according to the present invention had an excellent erythema relieving effect.

## Claims

1. A sphingolipid having a branched chain fatty acid and represented by chemical formula 1 below: 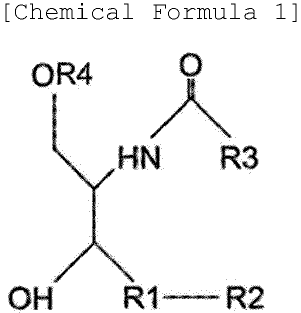 (wherein,
R1 is -CH₂-CH₂, -CH=CH, or -C(H)OH-CH₂;
R2 is a straight or branched chain alkyl, alkenyl, or alkynyl group of 12 to 20 carbon atoms;
R3 is a straight or branched chain alkyl, alkenyl, or alkynyl group of 12 to 24 carbon atoms, which may be optionally substituted with a hydroxyl group and in which two methyl groups are attached to at least one carbon atom; and
R4 is H or a straight or branched chain alkyl or alkenyl group of 1 to 10 carbon atoms).

2. The sphingolipid of claim 1, wherein the sphingolipid has a melting point of 10 to 100°C.

3. The sphingolipid of claim 1, wherein the sphingolipid has a melting point of 70 to 100°C.

4. A composition for application to the skin, the composition comprising the sphingolipid having a branched chain fatty acid and represented by Chemical Formula 1 of claim 1, cholesterol, a fatty acid, a medium chain triglyceride, and a phospholipid.

5. The composition of claim 4, further comprising a free sphingoid base, an organic acid, and water as balance to a total of 100 wt%.

6. The composition of claim 4, wherein the composition comprises 0.01 to 6 wt% of the sphingolipid, 0.1 to 5 wt% of cholesterol, 0.1 to 8 wt% of the fatty acid, 0.1 to 5 wt% of the medium chain triglyceride, and 0.1 to 5 wt% of the phospholipid.

7. The composition of claim 5, wherein the composition comprises 0.1 to 3 wt% of the free sphingoid base, 0.05 to 0.3 wt% of the organic acid, and water as balance to a total of 100 wt%.

8. The composition of claim 4, wherein the fatty acid is at least one selected from the group consisting of lauric acid, myristic acid, palmitic acid, stearic acid, oleic acid, arachidic acid, behenic acid, lignoceric acid, cerotic acid, arachidonic acid, eicosapentaenoic acid, erucic acid, branched chain lauric acid, branched chain myristic acids, branched chain palmitic acid, branched chain stearic acid, branched chain oleic acid, branched chain arachidic acid, branched chain behenic acid, and branched chain lignoceric acid.

9. The composition of claim 4, wherein the phospholipid is at least one selected from the group consisting of hydrogenated lecithin, hydrogenated phosphatidylcholine, phospholipids, hydrogenated lysophosphatidylcholine, hydrogenated lysolecithin, hydroxylated lecithin, and unsaturated lecithin.

10. The composition of claim 5, wherein the free sphingoid base is at least one selected from the group consisting of phytosphingosine, sphingosine, and sphinganine.

11. The composition of claim 4, further comprising at least one ceramide selected from the group consisting of ceramides 1, 2, 3, 4, 5, 6, 7, 8, and 9.

12. The composition of claim 4, wherein the composition prevents the adsorption of nitrates, sulfates, carbons, and other minerals, with a size of 0.1 to 100 µm, through the filling of the stratum corneum.

13. A cosmetic composition comprising the composition of claim 4 at 0.00001 to 50 wt% relative to the total weight thereof.

14. The cosmetic composition of claim 13, wherein the cosmetic composition is used to prevent fine dust adsorption.

15. The cosmetic composition of claim 13, wherein the cosmetic composition is used to moisturize the skin and enhance skin barriers.

16. The cosmetic composition of claim 13, wherein the cosmetic composition is used to relieve body wrinkles and modify the skin.

17. A pharmaceutical composition comprising the composition of claim 4 at 0.00001 to 50 wt% relative to the total weight thereof.

18. The pharmaceutical composition of claim 17, wherein the pharmaceutical composition is used to prevent or treat inflammation.

19. The pharmaceutical composition of claim 17, wherein the pharmaceutical composition is used to moisturize the skin and enhance skin barriers.

20. The pharmaceutical composition of claim 17, wherein the pharmaceutical composition is used to relieve body wrinkles and modify the skin.

21. A method for preparing a composition for application to the skin, the method comprising:
(1) preparing a lipid phase by dissolving, in an oil, a sphingolipid having a branched chain fatty acid and represented by Chemical Formula 1, cholesterol, a fatty acid, and a phospholipid;
(2) preparing an aqueous phase by mixing water with a mixture of a free sphingoid base and an organic acid; and
(3) mixing the lipid phase prepared in step (i) and the aqueous phase prepared in step (ii) under heating.

22. The method of claim 21, wherein in step (1), the fatty acid is at least one selected from the group consisting of lauric acid, myristic acid, palmitic acid, stearic acid, oleic acid, arachidic acid, behenic acid, lignoceric acid, cerotic acid, arachidonic acid, eicosapentaenoic acid, erucic acid, branched chain lauric acid, branched chain myristic acids, branched chain palmitic acid, branched chain stearic acid, branched chain oleic acid, branched chain arachidic acid, branched chain behenic acid, and branched chain lignoceric acid.

23. The method of claim 21, wherein in step (1), the phospholipid is at least one selected from the group consisting of hydrogenated lecithin, hydrogenated phosphatidylcholine, phospholipids, hydrogenated lysophosphatidylcholine, hydrogenated lysolecithin, hydroxylated lecithin, and unsaturated lecithin.

24. The method of claim 21, wherein in step (1), a medium chain triglyceride is further dissolved in the oil.

25. The method of claim 21, wherein in step (2), the free sphingoid base is at least one selected from the group consisting of sphingosine, phytosphingosine, and sphinganine.
